(19) Europäisches Patentamt / European Patent Office / Office européen des brevets

(11) EP 3 041 457 B1

(12) **EUROPEAN PATENT SPECIFICATION**

(45) Date of publication and mention of the grant of the patent:
**22.03.2017 Bulletin 2017/12**

(21) Application number: **14752348.4**

(22) Date of filing: **18.08.2014**

(51) Int Cl.:
***A61K 8/36*** (2006.01) ***A61K 8/39*** (2006.01)
***A61K 8/42*** (2006.01) ***A61Q 15/00*** (2006.01)
***A61K 8/92*** (2006.01) ***A61K 8/02*** (2006.01)

(86) International application number:
**PCT/EP2014/067596**

(87) International publication number:
**WO 2015/028340 (05.03.2015 Gazette 2015/09)**

# (54) PROCESS TO MANUFACTURE ANTIPERSPIRANT COMPOSITIONS

VERFAHREN ZUR HERSTELLUNG SCHWEISSHEMMENDER ZUSAMMENSETZUNGEN

PROCÉDÉ DE FABRICATION DE COMPOSITIONS ANTITRANSPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.09.2013 EP 13182560**

(43) Date of publication of application:
**13.07.2016 Bulletin 2016/28**

(73) Proprietors:
- **Unilever PLC**
  **London, Greater London EC4Y 0DY (GB)**
  Designated Contracting States:
  **CY GB IE MT**
- **Unilever NV**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
- **CIAMPI, Elisabetta**
  **Leeds**
  **Yorkshire LS14 2AR (GB)**
- **MARRIOTT, Robert Edward**
  **Wirral**
  **Merseyside CH63 3JW (GB)**
- **ROBERTS, Louise Jannette**
  **Wirral**
  **Merseyside CH63 3JW (GB)**
- **TROW, Victoria Louise**
  **Leeds**
  **Yorkshire LS14 2AR (GB)**
- **WHITEHEAD, Emma Jayne**
  **Wirral**
  **Merseyside CH63 3JW (GB)**

(74) Representative: **McHugh, Paul Edward**
**Unilever PLC**
**Unilever Patent Group**
**Colworth House**
**Sharnbrook**
**Bedford**
**Bedfordshire MK44 1LQ (GB)**

(56) References cited:
**EP-A2- 1 813 310**
**WO-A1-2008/145582**
**GB-A- 2 431 580**
**US-A1- 2012 276 033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

This invention relates to a process to manufacture an antiperspirant composition. More particularly, the invention is directed to a process to manufacture solid cosmetic antiperspirant compositions in the form of a stick.

### BACKGROUND OF THE INVENTION

Antiperspirant compositions are applied to the underarm area. It is desirable to include ingredients that provide a skin care benefit to this underarm area. The difficulty with formulating antiperspirant products to provide a skin care benefit is that many skin care benefit agents from the cosmetics field are difficult to formulate into antiperspirant products due to incompatibility with common ingredients in antiperspirants, such as the antiperspirant active, or have negative interactions such that sensory problems are introduced into the product.
One such class of ingredients is the hydroxyalkyl ureas.

WO 2008/145582 A1 discloses on page 15 an antiperspirant incorporating hydroxyethyl urea in combination with glycerol.

A problem with inclusion of this class of materials is that the resulting product feels gritty to the user, which is undesirable for a product that is applied to the skin.

We have found that a stable non-gritty solid antiperspirant product containing a hydroxyalkyl urea can be provided by application of specific process steps.

### SUMMARY OF THE INVENTION

In a first aspect, the invention is directed to process of manufacture of a solid cosmetic antiperspirant composition in the form of a stick comprising from 0.0005 to 3 wt.% of a hydroxyalkyl urea, the process comprising the steps of:-

(a) providing a stick dispenser comprising a stick barrel;
(b) providing a first mixture comprising a solvent and gellant and heating the mixture to a temperature above the melting point of the gellant;
(c) providing a second mixture comprising a hydroxyalkyl urea and a carrier material selected from the group consisting of: a natural oil that comprises a triglyceride of an unsaturated carboxylic acid, and polyalkylene glycols having a molecular weight of up to 650 Daltons, and heating this mixture to a temperature of 65°C or above;
(d) mixing together of the first and second mixtures;
(e) addition of an antiperspirant active to the mixture (d); and,
(f) filling a stick barrel with the mixture (e).

Preferably the first mixture is heated to a temperature of from 65°C to 120°C, preferably 70°C to 100°C, more preferably 80°C to 95°C

Preferably for step d), each of the two mixtures is at a temperature of 60°C to 100°C, preferably 65°C to 90°C, more preferably 75°C to 85°C.

Preferred hydroxyalkyl ureas have the following formula:

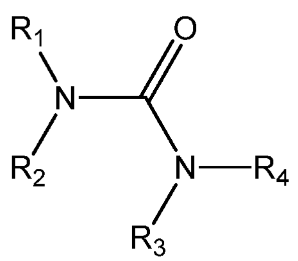

wherein $R_1$, $R_2$, $R_3$ and $R_4$ each independently represent: Hydrogen, a $C_{1-4}$ alkyl, or a $C_{2-6}$ hydroxyalkyl group, with the proviso that at least one of the groups $R_1$, $R_2$, $R_3$ and $R_4$ is a $C_{2-6}$ hydroxyalkyl group.

A particularly preferred hydroxyalkyl urea is a hydroxyethyl urea of structure:

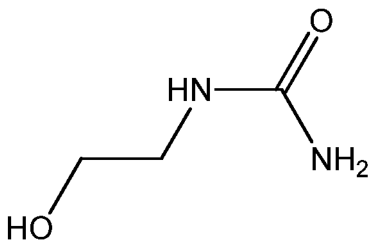

Preferably the natural oil comprises a triglyceride of an unsaturated carboxylic acid containing 1, 2, or 3 olefinic bonds.

Preferably the natural oil comprises a triglyceride of an unsaturated carboxylic acid containing from 14 to 22 carbon atoms, preferably 16-20 carbon atoms, more preferably 18 carbons

Preferably the natural oil comprises sunflower seed oil.

Preferably the polyalkylene glycol carrier material is selected from the group consisting of: polybutylene glycol, polypropylene glycol, polyethylene glycol and mixtures thereof.

The polyalkylene glycol carrier material has a molecular weight of up to 650 Daltons, preferably from 360 to 460 Daltons.

Preferably the carrier material is selected from: sunflower seed oil; and, polyethylene glycol having a molecular weight of from 360 to 460 Daltons and mixtures thereof.

Preferably the antiperspirant stick composition comprises no more than 1 wt.% of water, more preferably the stick composition is anhydrous.

Preferably the hydroxyalkyl urea is included at a level of from the hydroxyalkyl urea is included at a level of from 0.0005 to 2.5 wt.%, more preferably from 0.001 to 2 wt.%.

A preferred antiperspirant active comprises an aluminium salt, preferably an aluminium/zirconium tetrachlorohydrex glycine complex.

The invention further relates in a second aspect to a solid antiperspirant stick obtained by the process of the first aspect of the invention.

## DETAILED DESCRIPTION OF THE INVENTION

As used herein, the term "comprising" means including, made up of, composed of, consisting and/or consisting essentially of.

All percentages quoted are wt.% based on total amount in the composition unless otherwise stated.

The invention is based on the finding that a stable non-gritty solid antiperspirant stick product containing a hydroxyalkyl urea can be provided by way of the process steps outlined herein.

### Antiperspirant Active

The composition comprises from 0.1 to 40 wt.%, preferably from 5 to 40 wt.% of antiperspirant active. Antiperspirant active is preferably incorporated in an amount of at least 5 wt.%, more particularly from 20 to 30 wt.% of the composition. More preferably the antiperspirant active is present in an amount of from 22 to 27 wt.% of the composition.

Antiperspirant actives for use herein are often selected from astringent active salts, including, in particular, aluminium, zirconium and mixed aluminium/zirconium salts, including both inorganic salts, salts with organic anions and complexes. Preferred astringent salts include aluminium, zirconium and aluminium/zirconium halides and halohydrate salts, such as chlorohydrates and activated aluminium chlorohydrates.

Aluminium halohydrates are usually defined by the general formula $Al_2(OH)_xQ_y{\bullet}wH_2O$ in which Q represents chlorine, bromine or iodine, x is variable from 2 to 5 and x+y=6 while $wH_2O$ represents a variable amount of hydration. Zirconium actives can usually be represented by the empirical general formula: $ZrO(OH)_{2n-nz}B_z{\bullet}wH_20$ in which z is a variable in the range of from 0.9 to 2.0 so that the value 2n-nz is zero or positive, n is the valency of B, and B is selected from the group consisting of chloride, other halide, sulfamate, sulfate and mixtures thereof. Possible hydration to a variable extent is represented by $wH_2O$. In one embodiment B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Aluminium zirconium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula $CH_2(NH_2)COOH$.

It is highly desirable to employ complexes of a combination of aluminium halohydrates and zirconium chlorohydrates together with amino acids such as glycine, examples of which are disclosed in U.S. Pat. No. 3,792,068 (Luedders et al).

Certain of those Al/Zr complexes are commonly called AZG in the literature. AZG actives generally contain aluminium, zirconium and chloride with an Al/Zr ratio in a range from 2 to 10, especially 2 to 6, an Al/Cl ratio from 2.1 to 0.9 and a variable amount of glycine. Actives of this type are available from suppliers that include Summit Reheis. In one preferred embodiment the active is enhanced activity or activated aluminium/zirconium halohydrate, in particular, activated aluminium-zirconium tetrachlorohydrex glycine (AAZG).

Other actives which may be utilized include astringent titanium salts, for example those described in GB 2299506A.

The proportion of solid particulate antiperspirant salt in a suspension composition normally includes the weight of any water of hydration and any complexing agent that may also be present in the solid active.

In one or more embodiments it is desirable that the mean particle size of the antiperspirant salts is within the range of 0.1 to 100 micron with a mean particle size that is often from 3 to 30 microns, more particularly from 5 to 35 microns, and certain embodiments of interest from 10 to 25 microns.

The particulate antiperspirant active may be present in the form of hollow spheres or dense particles (by which is meant particles which are not hollow) at the discretion of the manufacturer. To reduce the appearance of visible deposits on the skin to which the composition is applied or on clothing which comes into contact with the composition, it is preferable for the particles to be substantially free from hollows. Hollows can be eliminated by crushing the spheres.

The most preferred antiperspirant salt is an aluminium/zirconium tetrachlorohydrex glycine complex.

Hydroxyalkyl Urea

Hydroxyalkyl ureas may be derived from urea ($NH_2CONH_2$).

Preferred hydroxyalkyl ureas have the formula:

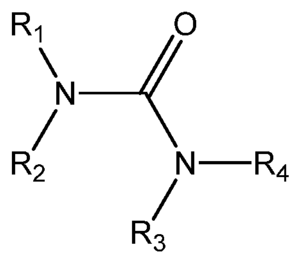

wherein $R_1$, $R_2$, $R_3$ and $R_4$ each independently represent: Hydrogen, a $C_{1-4}$ alkyl, or a $C_{2-6}$ hydroxyalkyl group, with the proviso that at least one of the groups $R_1$, $R_2$, $R_3$ and $R_4$ is a $C_{2-6}$ hydroxyalkyl group.

A particularly preferred hydroxyalkyl urea is hydroxyethyl urea, of structure:

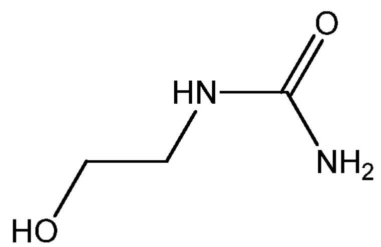

The hydroxyalkyl urea is present at a level of from 0.0005 to 3 wt.%, preferably from 0.0005 to 2.5 wt.%, more preferably from 0.001 to 2 wt.%, most preferably from 0.01 to 2 wt.%.

Preferably the hydroxyalkyl urea is included is included as a premix with the carrier material.

Carrier Material

The carrier material is selected from the group consisting of: a natural oil that comprises a triglyceride of an unsaturated carboxylic acid, and polyalkylene glycols having a molecular weight of up to 650 Daltons.
Preferably the natural oil comprises a triglyceride of an unsaturated carboxylic acid containing 1, 2, or 3 olefinic bonds.
Preferably the natural oil comprises a triglyceride of an unsaturated carboxylic acid containing from 14 to 22 carbon atoms, preferably 16-20 carbon atoms, more preferably 18 carbon atoms.
Preferably the natural oil comprises sunflower seed (*Helianthus annuus*) oil.
Preferably the polyalkylene glycol carrier material is selected from the group consisting of: polybutylene glycol, polypropylene glycol, polyethylene glycol and mixtures thereof.
The polyalkylene glycol carrier material has a molecular weight of up to 650 Daltons, preferably from 360 to 460 Daltons.
Preferably the carrier material is selected from: sunflower seed oil, polyethylene glycol having a molecular weight of from 360 to 460 Daltons and mixtures thereof.
The preferred level of carrier material present in the composition is from 0.1 to 10 wt.%, preferably from 0.5 to 7.5 wt.%
A preferred ratio of hydroxyalkyl urea to carrier material is from 1:5 to 2:1, more preferably 1:2.5 to 1:1.

Form of the Invention

The process is for the manufacture of a solid antiperspirant composition in the form of a stick.
Preferably the stick composition comprises no more than 1 wt.% of water, more preferably the stick composition is anhydrous.
The stick composition is solid, in that it retains its shape in the form of a stick without lateral support. The solid form is usually provided by way of a gellant.
The hardness of solid sticks is commonly measured using a conventional penetrometer test in which a Seta needle weighing 50g and having a tip angle of 9° 10' +/- 15' is allowed to drop for 5 seconds from surface contact with the test material. Desirably, the needle penetrates less than 30mm, preferably less than 20mm and especially up to 15mm, by virtue of the concentration of gellant or gellant mixture employed to solidify the composition.
An alternative method of measuring hardness employs a Stable Micro Systems TA.XT2i Texture Analyzer and Texture Expert Exceed™ software to generate the motion profile of a spherical probe employed in the method. A specific protocol involves a metal sphere, of diameter 9.5mm, is attached to the underside of a 5kg load cell, and positioned just above the sample surface. Under control of Expert Exceed™ software, the sphere is indented into the sample at an indentation speed of 0.05mm/s for a distance of 7 mm and reversed to withdraw the sphere from the sample at the same speed. Data comprising time(s) distance (mm) and force (N) is acquired at a rate of 25 Hz. The hardness H at a penetration of 4.76 mm is calculated using the formula:

$$H=F/A$$

in which H is expressed in $N/mm^2$, F is the load at the same travelled distance in N, and A is the projected area of the indentation in $mm^2$. This area can be calculated geometrically and is equal to the area of a diametral plane of the sphere, i.e., $\pi \times (4.76)^2 mm^2$. A measured hardness of at least $0.5N/mm^2$ indicates a solid stick.
Preferred gelling agents comprise: from 10 to 25 wt.% of a fatty alcohol wax; and, from 1 to 15 wt.% of hydrogenated castor oil.
In the stick composition, one or more further ingredients may be included, such as further oils, non-volatile emollient oil, silicone elastomers, gellants and fragrances.
Suitable solvents include silicone oils, which may be volatile or non-volatile. Particularly useful volatile oils are linear siloxanes containing from 3 to 9 silicon atoms, and cyclic siloxanes having from 4 to 6 silicon atoms, commonly referred to as cyclomethicone (e.g. cyclopentasiloxane, known as D5). Examples of commercially available volatile silicone oils include oils having grade designations 344, 345, 244, 245 and 246 from Dow Corning Corporation.
Particularly useful non-volatile silicone oils are polyalkyl siloxanes. Commercially available non-volatile silicone oils include products available under the trademarks Dow Corning 556 and Dow Corning 200 series
Solvents may be included at levels of from 5 to 75 wt.%, preferably 10 to 60 wt.%, more preferably 15 to 55 wt.%, or even 18 to 50 wt.%.
Suitable non-volatile emollient oils include the non-volatile silicone oils as previous described. Preferred non-volatile emollient oils are hydrocarbon oils, ester oils and ether oils.

Particularly preferred non-volatile emollient oils are $C_8$ to $C_{18}$ alkyl benzoate esters and PPG-14 butyl ether.

Non-volatile emollients, if present, may be present at a level of from 5 to 35 wt.%, preferably from 10 to 25 wt.%.

Silicone elastomers otherwise known as crosslinked dimethicone may be included, and are particularly useful in soft solid compositions,. Elastomers tend to thicken oils, often by absorbing them and swelling. The elastomer is typically crosslinked by reacting a silicone hydride with an $\alpha$-$\omega$ olefinically unsaturated dialkylene. If present, the elastomer is advantageously present at a concentration of at least 0.1 % up to 8%, and especially from 0.5% to 5% by weight of the antiperspirant composition. Elastomers are commercially available, for example from Dow Corning Inc and Shinetsu, and typically are supplied in a carrier oil that is frequently a cyclomethicone.

Preferred gellants include fatty alcohols having a melting range of from 55 to 75°C, and in many desirable embodiments, in the range of from 58 to 73°C. One or a blend of fatty alcohols can be employed, such as cetyl alcohol, stearyl alcohol, eicosyl alcohol and behenyl alcohol, or mixtures of any two or more thereof. Commercial fatty alcohols, though nominally and predominantly one specified alcohol often comprise a minor fraction, such as up to 5 or 6% by weight in total, of homologues differing by 2, 4 or even 6 carbons. When present, fatty alcohol is preferably present at a level of from 10 to 30 wt.%, preferably 11 to 20 wt.%, more preferably from 12.5 to 18.5 wt.%

Alternative gellants include waxes, preferably having a melting point in the range of from 70 to 95°C and especially from 75 to 90°C. Such waxes are often selected from hydrocarbon waxes and ester waxes, that can be derived from natural sources or synthesised. Suitable hydrocarbon waxes include mineral wax, microcrystalline wax, Montana wax, paraffin wax, and low molecular weight polyethylene, such as from 300 to 600 Daltons. Suitable ester waxes can be derived from unsaturated natural oils, such as plant-originating triglyceride oils by hydrogenation and optionally dehydroxylation (where the substituent contains at least one hydroxyl group as in castor oil). Suitable ester waxes include castor wax, candelilla wax, carnauba wax, beeswax and spermeceti wax. Natural waxes such as beeswax include a range of different chemical classes. Synthetic esters often comprise aliphatic monoesters containing at least 30 carbons, and indeed may be isolated natural products such as beeswax, or be derived from them or be the same compounds.

For solid sticks, a highly preferred gelling system is based on a fatty alcohol with a co-gellant wax as described above. Preferably the fatty alcohol would be present at a level of from 11 to 20 wt.%, and the wax would be present at a level of from 2 to 8 wt.%.

The most preferred gellant system for solid sticks is to use stearyl alcohol as the fatty alcohol in combination with hydrogenated castor oil as the wax co-gellant.

The composition can contain as perfume: free fragrance, a profragrance, encapsulated fragrance or fragrance that is associated with a host substrate such as cyclodextrin, or a mixture of any two or more of such perfume options.

Perfume (fragrance) if present, may be present at a level of from 0.25 to 2.5 wt.%.

Dispensers

The compositions produced herein are suitable for dispensing from known cosmetic dispensers for solid antiperspirant formulations such as stick or soft solid dispensers. Such dispensers commonly comprise a barrel, often of round or oval transverse cross section, having an opening at a first end through which the composition is dispensed and an elevator at an opposed second end that can be advanced towards the first end. The elevator fits within the barrel. Commonly, the first end can be covered with a cap, conveniently dimensioned to push it over the exterior of the barrel.

For sticks, the opening is the full cross section of the barrel. The elevator can be advanced by insertion of finger within the barrel or by co-operation between a threaded spindle and aperture in the elevator, the spindle being rotated by either an externally protruding rotor wheel or by a pawl arrangement. Suitable dispensers for firm sticks are described, for example in US 4,232,977,
US 4, 605,330, WO 09 818695, WO 09 603899, WO 09 405180, WO 09 325113, WO 09 305678, EP 1 040 445, US 5,997,202, US 5,897,263, US 5,496,122,
US 5,275,496, US 6,598,767, US 6,299,369, or WO 2002/03830.

## Examples

Examples according to the invention are denoted with a number; examples not according to the invention are denoted with a letter.

Incorporation of Hydroxyethyl Urea (HEU) in a Solid Stick

The hydroxyethyl urea was sourced from Hydrovance as a 45-55% weight active in aqueous solution. Before use, it was freeze dried to yield a solid, which was then used in the formulations.

Table 1 - Base Formulation

| Ingredient (wt.%) INCI Name | Trade Name | Supplier | Base Stick |
|---|---|---|---|
| **Cyclomethicone** | PMX-0245 | Xiameter | Balance to 100% |
| **Aluminium Zirconium Tetrachlorohydrex Gly** | Reach 908 | Summit Reheis | 20.00 |
| **Stearyl Alcohol** | Lanette C18 Deo | Cognis | 17.00 |
| **$C_{12-15}$ Alkyl Benzoate** | Finsolv TN | Finetex / Innospec | 15.00 |
| **PPG-14 Butyl Ether** | Fluid AP | Dow Chemical | 9.00 |
| **Hydrogenated Castor Oil** | Castorwax MP80 | Vertellus Performance Materials | 4.00 |
| **Parfum** | | Givaudan | 1.20 |
| **Dimethicone** | DC 200 50cs PMX 200 50cs | Dow Corning Xiameter | 1.00 |
| **Polyethylene** | Performalene 400 | New Phase Technologies | 0.75 |
| **Silica** | Aerosil 200 | Evonik | 0.75 |
| **Steareth 100** | Brij S100 | Croda | 0.45 |
| **BHT** | Tenox BHT | Eastman | 0.05 |
| **PEG - 8** | Polyglykol 400 | Clariant | 0-2 |
| **Sunflower Seed Oil** | Akosun | Aarhus Karlshamn | 0-0.5 |

The general method of preparation of the sticks was as follows:-

1. Oils (cyclomethicone, alkyl benzoate, PPG-14 butyl ether, dimethicone, optional PEG-8 if included) and BHT were added together into an appropriate vessel, silica then added;

2. Contents of vessel were sheared until homogeneous (e.g. 7000rpm for 5 minutes);

3. Stearyl alcohol, wax, steareth 100 and polyethylene added to vessel;

4. Vessel heated to ca. 85°C to ensure complete dissolution of the waxes, and mixed (e.g. 250rpm using a Heidolph mixer) for 10 minutes;

5. Contents of vessel allowed to cool to 78°C, prior to slow addition of antiperspirant active. Contents of vessel mixed (e.g. 350rpm using a Heidolph mixer)

6. Temperature maintained above 70°C for at least 5 minutes following addition of all of the antiperspirant active

7. Fragrance (and optional sunflower seed oil if included) added at 65°C

8. Formulation poured directly into stick barrel at ca. 62°C and allowed to set.

Sticks made to the base formulation (both with and without optional PEG-8 and sunflower seed oil individually or together) using the above process proved to be non-gritty.

To these base stick forming process, attempts were made to incorporate hydroxyethyl urea (HEU), either alone - as a solid, or as a melt; or with a carrier material. The point of addition of the HEU in the process was also assessed. The sticks were assessed as to whether they formed a stable product, and if so, their level of grittiness.

Grittiness test

The resulting stick was tested for grit by a trained assessor. The assessment involved running an index finger over the domed surface of the stick formulation. The formulation was further assessed by removal of the domed surface of stick using a sharp knife, with the assessment involving running an index finger over the freshly cut stick surface of the stick formulation. These tests were repeated by running the stick over the back of the hand. The stick was then either assessed as gritty or non-gritty, and in some cases even lumpy. The gritty and lumpy sticks are unacceptable sticks for consumer use.

Addition of HEU

HEU was added as:-

1. Solid;
2. Melt (HEU alone);
3. Melt (with carrier material)

Solid HEU was heated up to ca. 85°C to provide HEU in a molten state prior to addition.

HEU was added at 4 different points in the process:-

A. With Oils (HEU added with oils in general method)
B. With Gellant (HEU added with gellant in general method)
C. After Gellant, but before Antiperspirant Active addition (HEU added after gellant/oil mixture is heated in step 4 in general method)
D. After Antiperspirant Active (HEU added after antiperspirant active added in in general method)

Table 1 - HEU addition alone, either as solid or as a melt

| | | | **Point of Addition** | | | | **12 week stability** | |
|---|---|---|---|---|---|---|---|---|
| **Exp Code** | **% HEU** | **HEU Form** | **A** with Oils | **B** with Gellant | **C** After Gellant/before Active | **D** After Active | **25°C** | **45°C** |
| **a** | 4 | Solid | X | | | | **Unstable** | **Unstable** |
| **b** | 2 | Solid | | X | | | **Gritty** | **Gritty** |
| **c** | 4 | Solid | | X | | | **Gritty** | **Gritty** |
| **d** | 4 | Solid | | | X | | **Lumps** | **Lumps** |
| **e** | 4 | Solid | | | | X | **Gritty** | **Gritty** |
| **f** | 4 | Solid | | | | X | **Gritty** | **Gritty** |
| **g** | 4 | Melt | | | X | | **Lumps** | **Lumps** |
| **h** | 4 | Melt | | | X | | **Lumps Gritty** | **Lumps** |
| **i** | 4 | Melt | | | | X | **Lumps** | **Gritty** |
| **j** | 2 | Melt | | | | X | **Gritty** | **Gritty** |
| **k** | 4 | Melt | | | | X | **Lumps** | **Gritty Lumps** |

These tests indicated that it was not possible to add HEU alone, either in the solid form; or as a melt to result in a stable stick that was not gritty or lumpy.

Table 2 - HEU addition as a melt with a carrier material

| | | | Point of Addition | | | | 12 week stability | |
|---|---|---|---|---|---|---|---|---|
| **Exp Code** | **% HEU** | **Carrier Material** | **A** with Oils | **B** with Gellant | **C** After Gellant/before Active | **D** After Active | **25°C** | **45°C** |
| **l** | 4 | 2% Glycerol | | | X | | **Gritty** | **Gritty Lumps** |
| **m** | 4 | 2% Glycerol | | | X | | **Gritty** | **Gritty** |
| **n** | 2 | 2% Glycerol | | | X | | **Gritty** | **Gritty** |
| **o** | 4 | 2% SSO | | | | X | **Lumps** | **Lumps** |
| **p** | 4 | 2% SSO | | | | X | **Lumps** | **Lumps** |
| **1** | 2 | 2% SSO | | | X | | **OK** | **OK** |
| **q** | 4 | 2% PEG-8 | | | X | | **Gritty** | **Gritty** |
| **2** | 2 | 2% PEG-8 | | | X | | **OK** | **OK** |
| **3** | 1.5 | 2% PEG-8 | | | X | | **OK** | **OK** |
| **4** | 1 | 2% PEG-8 | | | X | | **OK** | **OK** |
| **5** | 0.5 | 2% PEG-8 | | | X | | **OK** | **OK** |
| Glycerol has a Hildebrand solubility parameter of 16.3<br>PEG-8 has a Hildebrand solubility parameter of 11.3<br>Sunflower Seed Oil has a Hildebrand solubility parameter of ca. 8 | | | | | | | | |

These tests show that by incorporating HEU in a melt with a carefully selected carrier material, a stable, non-gritty stick can be made. Only materials with a Hildebrand solubility parameter of from 7 to 15 resulted in an acceptable non-gritty stick. Glycerol is outside this range, and resulted in an unacceptable gritty stick.

The melt must be added at a specific point in the process, namely after the addition of oils and gellant with mixing, but before the addition of antiperspirant active.

An example method of preparation of the sticks is as follows:-

1. Oils (cyclomethicone, alkyl benzoate, PPG-14 butyl ether, dimethicone) and BHT were added together into an appropriate vessel, silica then added;
2. Contents of vessel were sheared until homogeneous (e.g. 7000rpm for 5 minutes);
3. Stearyl alcohol, wax, steareth 100 and polyethylene added to vessel;
4. Vessel heated to ca. 85°C to ensure complete dissolution of the waxes, and mixed (e.g. 250rpm using a Heidolph mixer) for 10 minutes;
5. HEU added along with carrier material (triglyceride, polyalkylene glycol or a mixture thereof) and heated to ca. 85°C
6. Contents of vessel allowed to cool to 78°C, prior to slow addition of antiperspirant active. Contents of vessel mixed (e.g. 350rpm using a Heidolph mixer)
7. Temperature maintained above 70°C for at least 5 minutes following addition of all of the antiperspirant active
8. Fragrance added at 65°C
9. Formulation poured directly into stick barrel at ca. 62°C and allowed to set.

This process allows incorporation of HEU at levels of 0.0005 to 3 wt.% in a solid antiperspirant stick.

Table 3 - Stick examples according to the Invention

| Ingredient (wt.%) | 1 | 2 | 3 | 4 | 5 | 6 |
|---|---|---|---|---|---|---|
| **Cyclomethicone** (PMX-0245) | Balance to 100% | Balance to 100% | Balance to 100% | Balance to 100% | Balance to 100% | Balance to 100% |

(continued)

| Ingredient (wt.%) | 1 | 2 | 3 | 4 | 5 | 6 |
|---|---|---|---|---|---|---|
| **Aluminium Zirconium Tetrachlorohydrex Gly** (Reach 908) | 20.00 | 20.00 | 20.00 | 20.00 | 20.00 | 20.00 |
| **Stearyl Alcohol** (Lanette C18 Deo) | 17.00 | 17.00 | 17.00 | 17.00 | 17.00 | 17.00 |
| **$C_{12-15}$ Alkyl Benzoate** (Finsolv TN) | 15.00 | 15.00 | 15.00 | 15.00 | 15.00 | 15.00 |
| **PPG-14 Butyl Ether** (Fluid AP) | 9.00 | 9.00 | 9.00 | 9.00 | 9.00 | 9.00 |
| **Hydrogenated Castor Oil** (Castorwax MP80) | 4.00 | 4.00 | 4.00 | 4.00 | 4.00 | 4.00 |
| **Parfum** | 1.20 | 1.20 | 1.20 | 1.20 | 1.20 | 1.10 |
| **Dimethicone** (DC 200 50cs) (PMX 200 50cs) | 1.00 | 1.00 | 1.00 | 1.00 | 1.00 | 1.00 |
| **Polyethylene** (Performalene 400) | 0.75 | 0.75 | 0.75 | 0.75 | 0.75 | 0.75 |
| **Silica** (Aerosil 200) | 0.75 | 0.75 | 0.75 | 0.75 | 0.75 | 0.75 |
| **Steareth 100** (Brij S100) | 0.45 | 0.45 | 0.45 | 0.45 | 0.45 | 0.45 |
| **BHT** (Tenox BHT) | 0.05 | 0.05 | 0.05 | 0.05 | 0.05 | 0.05 |
| **HEU** (Hydrovance) | 2 | 2 | 2 | 1 | 0.5 | 0.001 |
| **PEG-8** (Polyglykol 400) | | 2 | 2 | 2 | 2 | 2 |
| **Sunflower Seed Oil** (Akosun) | 2 | | 0.5 | | 0.5 | 0.5 |

## Claims

**1.** A process of manufacture of a solid cosmetic antiperspirant composition in the form of a stick comprising from 0.0005 to 3 wt.% of a hydroxyalkyl urea, the process comprising the steps of:-

(a) providing a stick dispenser comprising a stick barrel;
(b) providing a first mixture comprising a solvent and gellant and heating the mixture to a temperature above the melting point of the gellant;
(c) providing a second mixture comprising a hydroxyalkyl urea and a carrier material selected from the group consisting of: a natural oil that comprises a triglyceride of an unsaturated carboxylic acid, and polyalkylene glycols having a molecular weight of up to 650 Daltons, and heating this mixture to a temperature of 65°C or above;
(d) mixing together of the first and second mixtures;
(e) addition of an antiperspirant active to the mixture (d); and,
(f) filling a stick barrel with the mixture (e).

**2.** A process according to claim 1, wherein the first mixture is heated to a temperature of from 65°C to 120°C, preferably

70°C to 100°C, more preferably 80°C to 95°C.

3. A process according to claim 1 or claim 2, wherein for step d), each of the two mixtures is at a temperature of 60°C to 100°C, preferably 65°C to 90°C, more preferably 75°C to 85°C.

4. A process according to any preceding claim wherein the hydroxyalkyl urea has a formula:

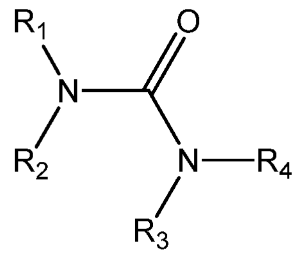

wherein $R_1$, $R_2$, $R_3$ and $R_4$ each independently represent: Hydrogen, a $C_{1-4}$ alkyl, or a $C_{2-6}$ hydroxyalkyl group, with the proviso that at least one of the groups $R_1$, $R_2$, $R_3$ and $R_4$ is a $C_{2-6}$ hydroxyalkyl group.

5. A process according to claim 4, wherein the hydroxyalkyl urea is a hydroxyethyl urea of structure:

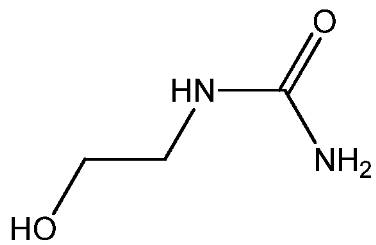

6. A process according to any preceding claim, wherein the natural oil comprises a triglyceride of an unsaturated carboxylic acid containing 1, 2, or 3 olefinic bonds.

7. A process according to claim 6, wherein the natural oil comprises a triglyceride of an unsaturated carboxylic acid containing from 14 to 22 carbon atoms, preferably 16-20 carbon atoms, more preferably 18 carbon atoms.

8. A process according to any preceding claim, wherein the natural oil comprises sunflower seed oil.

9. A process according to any preceding claim, wherein the polyalkylene glycol carrier material is selected from the group consisting of: polybutylene glycol, polypropylene glycol, polyethylene glycol and mixtures thereof.

10. A process according to any preceding claim, wherein the carrier material is selected from: sunflower seed oil, polyethylene glycol having a molecular weight of from 360 to 460 Daltons and mixtures thereof.

11. A process according to any preceding claim, wherein the resultant solid cosmetic antiperspirant composition comprises no more than 1 wt.% of water, preferably the stick composition is anhydrous.

12. A process according to any preceding claim, wherein the hydroxyalkyl urea is included at a level of from 0.0005 to 2.5 wt.%, more preferably from 0.001 to 2 wt.%.

13. A solid antiperspirant stick obtained by the process of any one of claims 1 to 12.

**Patentansprüche**

1. Verfahren zur Herstellung einer festen kosmetischen schweißhemmenden Zusammensetzung in Form eines Stiftes, der 0,0005 bis 3 Gew.-% eines Hydroxyalkylharnstoffs umfasst, wobei das Verfahren die Schritte umfasst:

(a) Bereitstellen einer Stiftausgabeeinrichtung, die einen Stiftzylinder umfasst,
(b) Bereitstellen einer ersten Mischung, die ein Lösungsmittel und ein Geliermittel umfasst, und Erhitzen der Mischung auf eine Temperatur oberhalb des Schmelzpunktes des Geliermittels,
(c) Bereitstellen einer zweiten Mischung, die einen Hydroxyalkylharnstoff und ein Trägermaterial umfasst, das aus der aus einem natürlichen Öl, das ein Triglycerid einer ungesättigten Carbonsäure umfasst, und Polyalkylenglykolen eines Molekulargewichts von bis zu 650 Dalton bestehenden Gruppe ausgewählt wird, und Erhitzen dieser Mischung auf eine Temperatur von 65°C oder darüber,
(d) Zusammenmischen der ersten und zweiten Mischung,
(e) Zugeben eines schweißhemmenden Wirkstoffs zu der Mischung (d) und
(f) Füllen eines Stiftzylinders mit der Mischung (e).

2. Verfahren nach Anspruch 1, wobei die erste Mischung auf eine Temperatur von 65°C bis 120°C, vorzugsweise von 70°C bis 100°C, bevorzugter von 80°C bis 95°C erhitzt wird.

3. Verfahren nach Anspruch 1 oder Anspruch 2, wobei für Schritt (d) jede der zwei Mischungen bei einer Temperatur von 60°C bis 100°C, vorzugsweise von 65°C bis 90°C, bevorzugter von 75 bis 85°C, vorliegt.

4. Verfahren nach irgendeinem vorhergehenden Anspruch, wobei der Hydroxyalkylharnstoff eine Formel

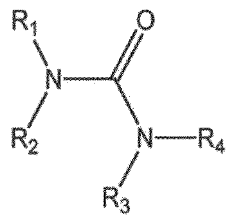

hat, wobei $R_1$, $R_2$, $R_3$ und $R_4$, jeweils unabhängig, darstellen: Wasserstoff, eine $C_{1-4}$-Alkyl- oder eine $C_{2-6}$-Hydroxyalkylgruppe, mit der Maßgabe, dass mindestens eine der Gruppen $R_1$, $R_2$, $R_3$ und $R_4$ eine $C_{2-6}$-Hydroxyalkylgruppe ist.

5. Verfahren nach Anspruch 4, wobei der Hydroxyalkylharnstoff ein Hydroxyethylharnstoff der Struktur

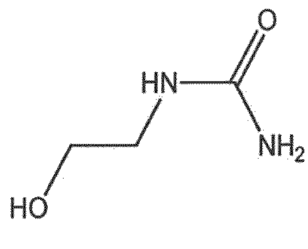

ist.

6. Verfahren nach irgendeinem vorhergehenden Anspruch, wobei das natürliche Öl ein Triglycerid einer ungesättigten Carbonsäure, die 1, 2 oder 3 olefinische Bindungen enthält, umfasst.

7. Verfahren nach Anspruch 6, wobei das natürliche Öl ein Triglycerid einer ungesättigten Carbonsäure umfasst, die 14 bis 22 Kohlenstoffatome, vorzugsweise 16 bis 20 Kohlenstoffatome, bevorzugter 18 Kohlenstoffatome, enthält.

8. Verfahren nach irgendeinem vorhergehenden Anspruch, wobei das natürliche Öl Sonnenblumenöl umfasst.

9. Verfahren nach irgendeinem vorhergehenden Anspruch, wobei das Polyalkylenglykol-Trägermaterial aus der Gruppe ausgewählt wird, die aus Polybutylenglykol, Polypropylenglykol, Polyethylenglykol und Mischungen davon besteht.

10. Verfahren nach irgendeinem vorhergehenden Anspruch, wobei das Trägermaterial unter Sonnenblumenöl, Polyethylenglykol mit einem Molekulargewicht von 360 bis 460 Dalton und Mischungen davon ausgewählt wird.

**11.** Verfahren nach irgendeinem vorhergehenden Anspruch, wobei die anfallende feste kosmetische schweißhemmende Zusammensetzung nicht mehr als 1 Gew.-% Wasser umfasst, wobei die Stiftzusammensetzung vorzugsweise wasserfrei ist.

**12.** Verfahren nach irgendeinem vorhergehenden Anspruch, wobei der Hydroxyalkylharnstoff in einer Konzentration von 0,0005 bis 2,5 Gew.-%, bevorzugter von 0,001 bis 2 Gew.-%, einbezogen wird.

**13.** Fester schweißhemmender Stift, erhalten durch das Verfahren nach irgendeinem der Ansprüche 1 bis 12.

## Revendications

**1.** Procédé de fabrication d'une composition d'antiperspirant cosmétique solide dans la forme d'un stick comprenant de 0,0005 à 3 % en masse d'une hydroxyalkylurée, le procédé comprenant les étapes de :

(a) fourniture d'un distributeur de stick comprenant un étui de stick ;
(b) fourniture d'un premier mélange comprenant un solvant et un gélifiant et chauffage du mélange à une température supérieure au point de fusion du gélifiant ;
(c) fourniture d'un second mélange comprenant une hydroxyalkylurée et un matériau de support choisi dans le groupe constitué de : une huile naturelle qui comprend un triglycéride d'un acide carboxylique insaturé, et des polyalkylèneglycols ayant une masse moléculaire jusqu'à 650 Daltons, et chauffage de ce mélange à une température de 65°C ou supérieure ;
(d) mélange ensemble des premier et second mélanges ;
(e) addition d'un actif d'antiperspirant au mélange (d) ; et,
(f) remplissage d'un étui de stick avec le mélange (e).

**2.** Procédé selon la revendication 1, dans lequel le premier mélange est chauffé à une température de 65°C à 120°C, de préférence de 70°C à 100°C, encore mieux de 80°C à 95°C.

**3.** Procédé selon la revendication 1 ou la revendication 2, dans lequel pour l'étape d), chacun des deux mélanges est à une température de 60°C à 100°C, de préférence de 65°C à 90°C, encore mieux de 75°C à 85°C.

**4.** Procédé selon l'une quelconque des revendications précédentes, dans lequel l'hydroxyalkylurée présente une formule :

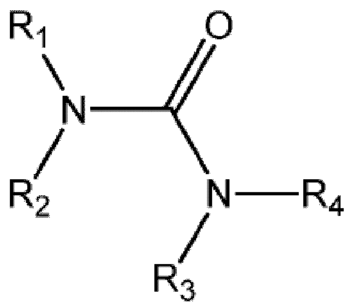

où $R_1$, $R_2$, $R_3$ et $R_4$ représentent chacun indépendamment :

l'hydrogène, un groupe alkyle en $C_{1-4}$ ou un groupe hydroxyalkyle en $C_{2-6}$ à condition qu'au moins un des groupes $R_1$, $R_2$, $R_3$ et $R_4$ soit un groupe hydroxyalkyle en $C_{2-6}$.

**5.** Procédé selon la revendication 4, dans lequel l'hydroxyalkylurée est une hydroxyéthylurée de structure :

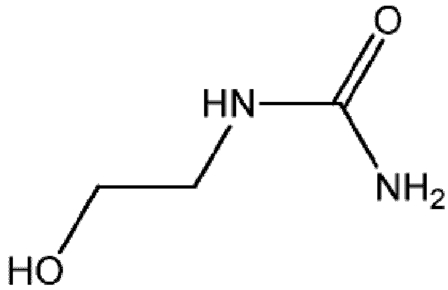

6. Procédé selon l'une quelconque des revendications précédentes, dans lequel l'huile naturelle comprend un triglycéride d'un acide carboxylique insaturé contenant 1, 2, 3 liaisons oléfiniques.

7. Procédé selon la revendication 6, dans lequel l'huile naturelle comprend un triglycéride d'un acide carboxylique insaturé contenant de 14 à 22 atomes de carbone, de préférence 16-20 atomes de carbone, encore mieux 18 atomes de carbone.

8. Procédé selon l'une quelconque des revendications précédentes, dans lequel l'huile naturelle comprend de l'huile de graines de tournesol.

9. Procédé selon l'une quelconque des revendications précédentes, dans lequel le matériau de support de polyalkylèneglycol est choisi dans le groupe constitué de : polybutylèneglycol, polypropylèneglycol, polyéthylèneglycol et mélanges de ceux-ci.

10. Procédé selon l'une quelconque des revendications précédentes, dans lequel le matériau de support est choisi parmi : l'huile de graine de tournesol, du polyéthylèneglycol ayant une masse moléculaire de 360 à 460 Daltons et des mélanges de ceux-ci.

11. Procédé selon l'une quelconque des revendications précédentes, dans lequel la composition d'antiperspirant cosmétique solide résultante comprend au plus 1 % en masse d'eau, la composition de stick est de préférence anhydre.

12. Procédé selon l'une quelconque des revendications précédentes, dans lequel l'hydroxyalkylurée est incluse à une teneur de 0,0005 à 2,5 % en masse, encore mieux de 0,001 à 2 % en masse.

13. Stick d'antiperspirant solide obtenu par le procédé selon l'une quelconque des revendications 1 à 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008145582 A1
- US 3792068 A, Luedders
- GB 2299506 A
- US 4232977 A
- US 4605330 A
- WO 09818695 A
- WO 09603899 A
- WO 09405180 A
- WO 09325113 A
- WO 09305678 A
- EP 1040445 A
- US 5997202 A
- US 5897263 A
- US 5496122 A
- US 5275496 A
- US 6598767 B
- US 6299369 B
- WO 200203830 A